# EUROPEAN PATENT APPLICATION

(11) **EP 4 609 892 A1**
(43) Date of publication of application: **03.09.2025**
(21) Application number: 24160905.6
(22) Date of filing: 01.03.2024
(51) Int. Cl.: A61M 3/02, B65D 1/00

(54) **CONTAINER FOR IRRIGATION APPLICATION PRODUCIBLE ON A PRODUCTION LINE FOR PRODUCING INFUSION CONTAINERS**

(71) Applicant: B. Braun Melsungen AG, 34212 Melsungen (DE)
(72) Inventor: LAMPRECHT, Volker, 34212 Melsungen (DE)
(74) Representative: Maiwald GmbH

(57) **Abstract**

A container for irrigation application comprising a bottom part, a cylindrical or conical lower body part comprising a lower bottom area and a lower top area, a first transition part, a cylindrical or conical middle body part comprising a middle bottom area and middle top area, a second transition part, and a cylindrical or conical upper body part comprising an upper bottom area, wherein the bottom part is fluidly connected with the lower body part via the lower bottom area, the first transition part is fluidly connected with the lower body part via the lower top area, the first transition part is fluidly connected with the middle body part via the middle bottom area, the second transition part is fluidly connected with the middle body part via the middle top area, and the second transition part is fluidly connected with the upper body part via the upper bottom area, wherein the lower bottom area, the lower top area, the middle bottom area, the middle top area, and the upper bottom area have an oval shape, and wherein the surface area of the middle bottom area is lower than the surface area of the lower top area and wherein the surface area of the middle top area is lower than the surface area of the upper bottom area.

## Description

### Technical Field of the Invention

The present invention is located in the field of containers for irrigation application, the use of such containers in irrigation application, and a method for irrigating an object during surgery with such a container.

### Background of the Invention

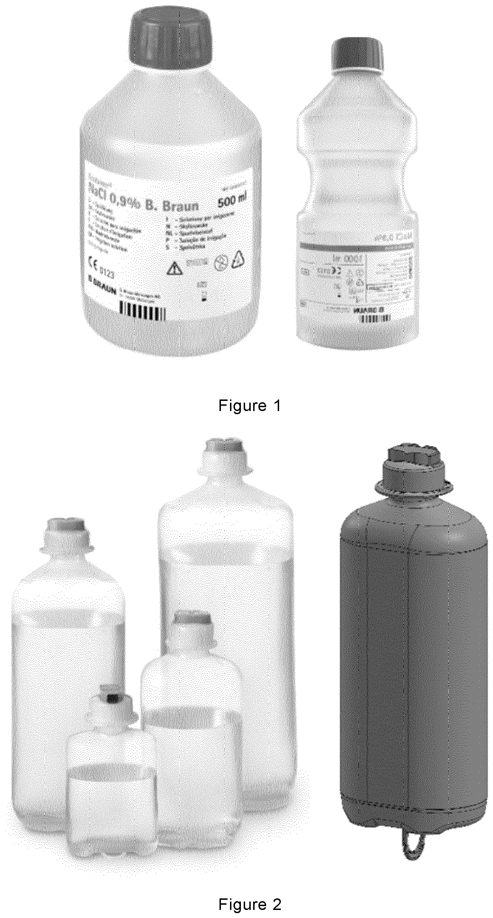

Irrigation containers are usually used to drain and wash surgery fields, such as body cavities, tissues, wounds, and burns, during surgeries. Typically, irrigation containers are bottles comprising a sterile liquid, wherein the bottle should be openable by hand (cf. Figure 1). Usually, during operation, the sterile liquid is poured out thereby ensuring that the sterile status of the liquid is kept intact up until the liquid contacts the surgery field. Nowadays, normally blown bottles made from plastic material are used to achieve this simple functionality. As irrigation bottles are usually emptied by hand either only by pouring or also additionally by applying pressure to the walls of the container, a collapsible shape would have the disadvantage that the handling of the irrigation container becomes problematic. Usually, irrigation containers have the shape of cylinders based on a base area having the shape of a circle. This ensures that the containers do not collapse during emptying. Hence, containers for irrigation application usually resemble normal water bottles and may have a recessed grip at the container walls.

Containers for infusion application, on the other hand, have usually rectangular-oval shaped base areas, since this geometry favors the collapsing behavior during the use for infusion of solutions and makes an approximately complete emptying of this container possible in the first place (cf. Figure 2). According to EP 1 663 100 A1, such containers for infusion application are usually manufactured without a recessed grip and have a suspension tab on the base.

Containers for irrigation application and containers of infusion application are produced on production lines, which comprise a molding machine including respective molds for the containers to be produced, preferably mold blowing machines. Furthermore, the produced containers are put into carriers, which are connected to a conveyor line, and transported to further machines such as machines for adding further parts, such as caps, machines for filling the containers with irrigation or infusion liquids, machines for labelling the containers, and machines for sterilizing the filled containers.

### Summary of the Invention

Currently, containers for irrigation application cannot be produced on a production line for producing containers for infusion solutions. Reason is that their shapes differ significantly due to their different requirements in view of collapsibility. Hence, for the production of both, containers for irrigation application and for infusion application, two production lines must be provided and maintained resulting in higher space requirements, higher maintaining costs etc. Alternatively, the production line can be modified. However, such modification has the effect that a container for infusion application cannot be produced on the production line anymore. Furthermore, the modification affords changing major parts of the production line resulting in further costs and time without production.

Hence, there is the need for a container for irrigation application, which can be produced on a production line, which can also produce containers for infusion application with minor or preferably no modification necessary.

As indicated above, containers for infusion application usually have a base area having an oval shape. Such a base area assures that the container is collapsible, e.g., changes its three-dimensional shape towards a flattened, more two-dimensional-like shape in that the opposite walls move together during emptying of the container. Thus, the molds of the production line are configured to produce such a shape, the carriers of the production line are configured to hold such a shape and each other machine is configured to handle containers with such a base shape.

It has now been found out that with the modification of the shape of a container having an oval-shaped base area, a collapsing of such container can be prevented. As a result, such a container is producible on a production line, which is also suitable for producing container for infusion application. Nevertheless, as the collapsing is prevented, such container can be used for irrigation applications.

Thus, it has been surprisingly found out in the present invention that above-mentioned object can be solved by a container for irrigation application comprising a bottom part, a cylindrical or conical lower body part comprising a lower bottom area and a lower top area, a first transition part, a cylindrical or conical middle body part comprising a middle bottom area and middle top area, a second transition part, and a cylindrical or conical upper body part comprising an upper bottom area, wherein the bottom part is fluidly connected with the lower body part via the lower bottom area, the first transition part is fluidly connected with the lower body part via the lower top area, the first transition part is fluidly connected with the middle body part via the middle bottom area, the second transition part is fluidly connected with the middle body part via the middle top area, and the second transition part is fluidly connected with the upper body part via the upper bottom area, wherein the lower bottom area, the lower top area, the middle bottom area, the middle top area, and the upper bottom area have an oval shape, and wherein the surface area of the middle bottom area is lower than the surface area of the lower top area and wherein the surface area of the middle top area is lower than the surface area of the upper bottom area.

It has been further found out that above-mentioned object of the present invention can be achieved by the use of the container according to the present invention in an irrigation application.

Furthermore, it has been found out that the object of the present invention is achieved by a process for producing a container according to the present invention, the process comprising the steps of molding, preferably blow molding, the container, filling the container with an irrigation liquid, closing the container, and sterilizing the container according to the present invention.

The container of the present invention has the advantage that it can be produced in a production line for container for infusion application, as although it has a base area having an oval shape, the remaining shape prevents collapsing during emptying the container. Furthermore, the container of the present invention has the advantage that if the properties of the container are selected to provide a collapsible container with a base area not having an oval shape, the respective container having an oval shape would allow bringing itself into an uncollapsed form after emptying and collapsing. To the contrary, the respective collapsible container with a base area not having an oval shape will keep the collapsed shape, thereby significantly complicating handling. For such a container, rotation about 90° and pressure on the non-collapsed parts of the walls would be necessary to be brought back into non-collapsed shape.

### Brief Description of the Drawings

- Figure 1: shows containers for irrigation application usually used in the prior art.
- Figure 2: shows containers for infusion application usually used in the prior art.
- Figure 3: shows a schematic view of a part of the container according to one embodiment of the present invention.
- Figure 4: shows a schematic view of a container according to one embodiment of the present invention.
- Figure 5: shows a schematic view of a container according to one embodiment of the present invention different from the embodiment as shown in Figure 4.

### Reference signs

The following reference signs are used throughout the description and the figures.
- **1**: bottom part
- **2**: lower body part
- **3**: lower bottom area
- **4**: lower top area
- **5**: first transition part
- **6**: middle body part
- **7**: middle bottom area
- **8**: middle top area
- **9**: second transition part
- **10**: upper body part
- **11**: upper bottom area
- **12**: at least one outlet
- **a**: curved area of the sigmoid function of the first transition part closer to the lower body part
- **b**: curved area of the sigmoid function of the first transition part closer to the middle body part
- **c**: curved area of the sigmoid function of the second transition part closer to the upper body part
- **d**: curved area of the sigmoid function of the second transition part closer to the middle body part

### Definitions

The term "*irrigation solution*" as used herein denotes a liquid used in irrigation application. Irrigation solutions have to be sterile. An irrigating solution is suitable for killing microorganisms, disrupting the biofilm on surgery objects, inactivating virulence factors such as endotoxin, dissolving pulp-tissue remnants, removing hard-tissue debris and the smear layer created during instrumentation or prevent their formation, providing lubrication for instruments, and being biocompatible. Usually, an irrigation solution is based on water. Optionally, it can be mixed with an adjuvant. Adjuvants may be sodium chloride, sodium hypochlorite, potassium chloride, calcium chloride, chlorhexidine, ethylenediamine tetraacetic acid, citric acid, etidronic acid, maleic acid, sodium lactate, or mixtures thereof. Most preferably, irrigation solutions are selected from the list consisting of 0.9% aqueous sodium chloride solution, Ringer's lactate solution, and distilled water.

The term "*surgery object*" as used herein denotes an object, preferably body part, which can be treated by surgery, wherein the body part is preferably selected from a group consisting of body cavities, tissues, wounds, bones, and burns.

The term "*irrigation*" or "*irrigation application*" as used herein denotes the process of mechanical cleansing of surgery objects during topical, intra- or postoperative surgical interventions using an irrigation solution. This is typically achieved by pouring the irrigation solution on the surgery object and rinsing it, which is also known as "*direct irrigation*"*.* Alternatively, such a process is achieved by pouring out liquid in an intermediate recipient (like a kidney bowl), from which the solution is then taken out with a syringe to be spread onto the object, which is also known as "*indirect irrigation*"*.* The goal of the irrigation is to clear a surgical object from, e.g., blood and tissue to keep the surgery object sterile, prevent infections, moisten the surgery object, wound tamponades, cloths, bandages, and dressings, cleansing of operating instruments and accessories, and/or make the object visible for the surgeon. It is understood that prior to and/or during the process of irrigation, the container comprising the irrigation solution has to be put aside, at least one time.

The term "*upright position*" as used herein denotes an orientation of the bag of the present invention in that each distance of each point in the base part of the bag to the center of the gravitational force is smaller than in any point of the upper part and optionally the top part of the bag.

The term "*used in an upright position*" as used herein in particular in connection with the irrigation application denotes an irrigation process, in which the container is used such that the container is in upright position when being put aside.

The term "*sterile*" as used denotes the status of an object having a significantly reduced number of bacteria and/or viruses on its surface to reduce the risk of an infection. In particular, the term "*sterile*" denotes an object or substance, which has a bioburden load of lower than 10⁻⁶. The bioburden load can be measured i.e., according to ISO 11737-1:2018.

The term "*sterilization*" as used herein denotes a method to destroy all forms of living microorganisms from a substance. As there is always a certain probability of at least one microorganism to survive such procedure, the aim of sterilization is the reduction of initially present microorganisms or other potential pathogens. Generally, sterilization is accepted to be achieved if the bioburden load of the substance of object to be sterilized is lower than 10⁻⁶. The bioburden load can be measured i.e., according to ISO 11737-1:2018. Sterilization can be achieved using several methods. In one sterilization process the object is heated up to at least 105 °C to achieve a sterile object.

Thereby, the object should not be deformed by the elevated temperature. Preferably, the heating step is performed in an autoclave. Filtration methods are also used to sterilize liquids, i.e., by using membrane filters, Seitz filters, and/or candle filters. Finally, sterilization can be achieved by indirect energy import into or onto the object, e.g., by ultrasonic waves, ultraviolet light, as well as by high energy particles (such as electrons, gamma- or X-rays).

The term "*liquid-tight*" as used herein denotes a quality of an object to function as a barrier for a liquid, preferably for a water-based liquid.

The term "*oval shape*" as used herein denotes a plane curve, which is simple, i.e., not self-intersecting, convex, and closed. Preferably, an oval shape has at least one symmetry axis, more preferably two symmetry axes, and most preferably two symmetry axes, wherein the angle between these two symmetry axes is a rectangular angle. Specific oval shapes are circles, ellipsoids, rectangles with quadrant corners, and stadiums.

The term "*rectangular-oval shape*" as used herein denotes a rectangle with quadrant corners or a stadium shape, preferably a stadium shape.

The term "*cylindrical shape*" as used herein denotes a shape of all points on all lines which are parallel to a given main axis and which pass through a fixed plane curve in a plane not parallel, preferably perpendicular, to the main axis, wherein the main axis preferably passes through the centroid of the fixed plane curve. The lines have a defined length, which defines the length of the cylindrical shape. Hence, in a cylindrical shape, the fixed plane curve is identical at both ends. Most preferably, the main axis of the fixed plane curve is the circular symmetry axis of the fixed plane curve.

The term "*cone*" as used herein denotes a cylindrical shape, in which the fixed plane curves at both ends are not identical and wherein each line pass through the first and the second fixed plane are not parallel to the main axis. Preferably, the main axes of both fixed planes are identical. In a preferred embodiment of the cone, the fixed curve having the smaller surface can be a point.

The term "*collapsible container*" as used herein denotes a container, which changes its shape during emptying a fluid enclosed in the container from the container. Usually, this occurs in that opposite walls of the container move towards each other thereby reducing the inner volume of the container. During the process of emptying the container usually no air is sucked through the opening of the container. Thus, the emptying process is a continuous and smooth process. On the other hand, an at least partially incollapsible container does not allow for at least two walls to move towards each other. Hence, during emptying a fluid enclosed by the container from the container, from time-to-time air is sucked through the opening of the container, thereby interrupting the emptying process.

### Detailed Description of the Invention

In the following, the present invention will be described in detail. Thus, the container of the present invention, the use of the container of the present invention, and the process for producing the container of the present invention are described in the following.

### Container

Figure 2 shows a representative of commercially available infusion solution containers. The central part of the container has only the necessary rigidity, which is needed to stand upright, thus achieving a collapse during emptying, which is necessary for the infusion application. A safe gripping and pouring of such a container is not possible, because the body of the container is too unstable for this. However, for irrigation application, such rigidity is necessary. The collapsibility of the container is usually achieved by a base area, which has an oval shape. Hence, for being producible by a production line for container for infusion application, the container for irrigation application according to the present invention must have an oval-shaped base area.

Is has now been found out that if a container for infusion application having a base area with an oval shape is modified in that at least a part of the middle part of the body has at least in one direction a smaller diameter than the upper and lower body parts, the rigidity of the container is enhanced such that collapsing during emptying is prevented.

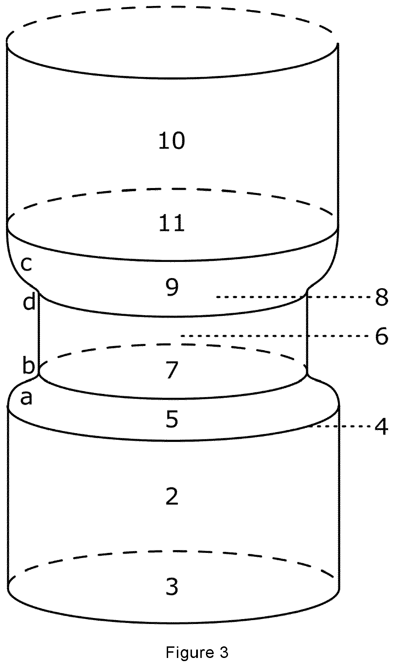

Hence, the container for irrigation application according to the most general embodiment of the present invention comprises (with reference to Figure 3)
- a bottom part (**1**),
- a cylindrical or conical lower body part (**2**) comprising a lower bottom area (**3**) and a lower top area (**4**),
- a first transition part (**5**),
- a cylindrical or conical middle body part (**6**) comprising a middle bottom area (**7**) and middle top area (**8**),
- a second transition part (**9**), and
- a cylindrical or conical upper body part (**10**) comprising an upper bottom area (**11**),

wherein the bottom part (**1**) is fluidly connected with the lower body part (**2**) via the lower bottom area (**3**), the first transition part (**5**) is fluidly connected with the lower body part (**2**) via the lower top area (**4**), the first transition part (**5**) is fluidly connected with the middle body part (**6**) via the middle bottom area (**7**), the second transition part (**9**) is fluidly connected with the middle body part (**6**) via the middle top area (**8**), and the second transition part (**9**) is fluidly connected with the upper body part (**10**) via the upper bottom area (**11**),
wherein the lower bottom area (**3**), the lower top area (**4**), the middle bottom area (**7**), the middle top area (**8**), and the upper bottom area (**11**) have an oval shape, and
wherein the surface area of the middle bottom area (**7**) is lower than the surface area of the lower top area (**4**) and wherein the surface area of the middle top area (**8**) is lower than the surface area of the upper bottom area (**11**).

Preferably, the oval shape is a shape selected from the list consisting of an ellipsoid, a rectangle with quadrant corners, and a stadium shape, more preferably is a stadium shape.

Preferably, the container of the present invention without the first transition part (**5**), without the cylindrical or conical middle body part (**6**), and/or without the second transition part (**9**) is a collapsible container. Hence, as soon as these features are removed from the base shape of the container according to the present invention, the shape is suitable for collapsing during emptying and, thus, suitable for infusion application.

It should be understood that the embodiment of Figure 3 does not show the most general embodiment according to the present invention. Generally, the first and second transition parts can have any shape, which allows for a constantly decreasing or increasing diameter. Hence, also linear gradients of the diameter are conceivable. Also generally, the diameter of the upper, the lower, and/or the middle body part are not necessarily constant, but could also be varying, i.e., having a conical shape.

Nevertheless, preferably, the shape of the lower body part (**2**), the middle body part (**6**), and/or the upper body part (**9**) is cylindrical or conical. The advantage of such an embodiment is that the space is used most economically, in particular when the containers are stored in staples. Even more preferably, the shape of the middle body part (**6**) is cylindrical. Such an embodiment has the advantage of having an improved grip. Preferably, the shape of the lower body part (**2**) and/or the upper body part (**10**) is cylindrical. Most preferably, the lower body part (**2**), the middle body part (**6**), and/or the upper body part (**9**) is cylindrical. This allows for best usage of space, best grip, and best handling during the production process.

Likewise, in the container according to the present invention the lower bottom area (**3**), the lower top area (**4**), and the upper bottom area (**11**) preferably have the same shape. This has the effect of a generally unique shape throughout the body. In particular if combined with a cylindrical shape. Hence, most preferably, in the container according to the present invention the lower bottom area (**3**), the lower top area (**4**), and the upper bottom area (**11**) have the same shape and the lower body part (**2**), the middle body part (**6**), and/or the upper body part (**9**) is cylindrical.

As set out above, generally, the first and second transition parts can have any shape, which allows for a constantly decreasing or increasing diameter, and, hence, also linear gradients of the diameter.

However, preferably, the shape of the first and/or the second transition parts (**5, 9**), more preferably the shape of the first and the second transition parts (**5, 9**), are formed as indicated in Figure 3. Such a shape allows for a secure and still convenient grip of the container. Hence, preferably, in the container of the present invention, the first transition part (**5**) is a cone, wherein in the cross section of the cone the shape of the rotational surface of the cone can be described by an asymmetric sigmoid function, wherein the change of slope in the curved area of the sigmoid function (**a**), which is closer to the lower body part (**2**), is smaller than the change of slope in the curved area of the asymmetric sigmoid function (**b**), which is closer to the middle body part (**6**).

Likewise, preferably, in the container according to the present invention, the second transition part (**9**) is a cone, wherein in the cross section of the cone the shape of the rotational surface of the cone can be described by an asymmetric sigmoid function, wherein the change of slope in the curved area of the sigmoid function (**c**), which is closer to the upper body part (**10**), is smaller than the change of slope in the curved area of the asymmetric sigmoid function (**d**), which is closer to the middle body part (**6**).

Most preferably, in the container according to the present invention the first transition part (**5**) is a cone, wherein in the cross section of the cone the shape of the rotational surface of the cone can be described by an asymmetric sigmoid function, wherein the change of slope in the curved area of the sigmoid function (**a**), which is closer to the lower body part (**2**), is smaller than the change of slope in the curved area of the asymmetric sigmoid function (**b**), which is closer to the middle body part (**6**), and the second transition part (**9**) is a cone, wherein in the cross section of the cone the shape of the rotational surface of the cone can be described by an asymmetric sigmoid function, wherein the change of slope in the curved area of the sigmoid function (**c**), which is closer to the upper body part (**10**), is smaller than the change of slope in the curved area of the asymmetric sigmoid function (**d**), which is closer to the middle body part (**6**).

It is preferable to have a generally base containers having circular symmetry, i.e., except for the grip recess part. This improves and facilitates handling by the production lines means. Hence, preferably, in the container according to the present invention, the main axes, preferably symmetry axes, of the lower top area (**4**) and the middle bottom area (**7**) are parallel and/or the main axes, preferably symmetry axes, of the upper bottom area (**11**) and the middle bottom area (**7**) are parallel. Most preferably, the main axes, preferably symmetry axes, of the lower top area (**4**), the middle bottom area (**7**), and the main axes, preferably symmetry axes, of the upper bottom area (**11**) are parallel.

In an especially preferred embodiment of the present invention, the symmetry axes of the lower bottom area (**3**), the lower top area (**4**), the middle bottom area (**7**), the middle top area (**8**), and/or the upper bottom area (**11**) are parallel, preferably identical.

Preferably, the height of the middle body part (**6**) is lower than the height of the lower body part (**2**), the height of the middle body part (**6**) is lower than the height of the upper body part (**10**), and/or the height of the lower body part (**2**) is larger than the height of the upper body part (**11**). More preferably, the height of the middle body part (**6**) is lower than the height of the lower body part (**2**), the height of the middle body part (**6**) is lower than the height of the upper body part (**10**), and the height of the lower body part (**2**) is larger than the height of the upper body part (**11**). This requires a shape, wherein the grip is above the center of gravity of the filled container. Thus, such a shape prevents unintended tilting of the container when held in hand during irrigation application.

An outlet comprised in the container of the present invention is not necessarily needed. It can be cut open before usage. However, preferably, the container according to the present invention comprises at least one outlet (12). More preferably, the upper body part of the container of the present invention comprises the least one outlet (**12**). This has the advantage that the handling of the container during irrigation application is facilitated and the whole volume of the solution comprised in the container can be emptied. Most preferably, the outlet is temporarily closed by an entity to avoid loss of irrigation solution during handling, but also to avoid contamination. Hence, the at least one outlet (**12**) is preferably selected from the list consisting of a tear-off opening, a spout, a twist-off opening, and a non-return valve.

Even more preferably, the at least one outlet (**12**) comprises a tamper-evidence feature.

The material used for the container is the material typically used for infusion and/or irrigation containers. Hence, preferably, the material of the container comprises, preferably consists of, a polymer material preferably a polymer material selected from the list consisting of polyethylene, polypropylene, polyethylene terephthalate, polyamide, ethylene vinyl alcohol, or mixture thereof. Such material provides the optimal balance between flexibility, rigidity, chemical inertness, and gas barrier properties.

Furthermore, it is advantageous if the skilled person can see from the outside how much solution is left in the container. Hence, preferably, the polymer material of the container of the present invention has a haze value measured according to ASTM D1003, Procedure B, of less than 30%, more preferably less than 20%, even more preferably less than 10%, and most preferably less than 5%. More preferably, the wall of the container according to the present invention has scale, more preferably a scale engraved into the wall, wherein the scale is suitable for measuring the volume of the liquid in the container.

Preferably, the polymer material of the container of the present invention has a flexural modulus in the range of from 400 to 800 MPa measured according to ISO 178. As also mentioned further below, such flexural modulus defines the optimal range for having a stiffness contributing best to collapsibility / incollapsibility properties.

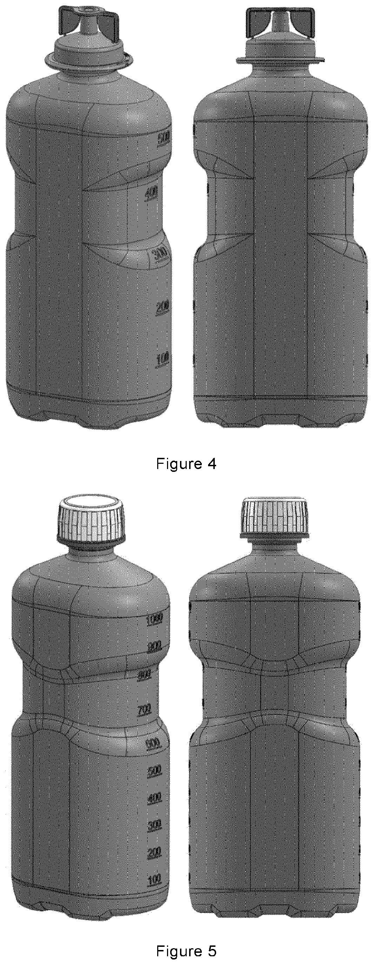

Figures 3 and 4 show containers according to a first and a second preferred embodiment of the present invention. The first preferred embodiment is concerned with a container for direct irrigation, while the second preferred embodiment is directed towards a container for indirect irrigation.

Figure 3 shows a container having basic dimensions according to the infusion solution container as shown in Figure 2. The difference is the partially recessed grip, which makes the body more rigid at the walls of the container and thus easier to grasp by hand. Thus, despite the unchanged basic shape, the collapsibility is replaced by grippability and the container thus becomes suitable for a different application compared to its origin. This improvement can be particularly observed for containers having an inner volume in the range of from 500 ml to 1000 ml. Hence, preferably, the inner volume of the container of the present invention is larger than 250 ml, more preferably larger than 350 ml, and most preferably at least 500 ml. Likewise, preferably, the inner volume of the container according to the present invention is smaller than 1500 ml, more preferably smaller than 1250 ml, and most preferably smaller than or equal to 1000 ml. Furthermore, this improvement can be in particular observed for containers having a wall thickness in the range of from 0.3 to 0.7 mm. Generally, different requirements result in different needs for wall thicknesses. Material reduction and thus sustainability requires a trend towards lower wall thicknesses. Preparation time is normally optimized in the direction of decreased preparation times thereby also causing the trend towards lower wall thicknesses. On the other hand, storage stability requires a trend towards higher wall thicknesses due to increased water permeability of lower wall thicknesses.

Furthermore, this improvement can be in particular observed for containers having a material having a flexural modulus in the range of from 400 to 800 MPa measured according to ISO 178. Most preferably, the container according to the present invention has an internal volume in the range of from 500 to 1000 ml, a flexural modulus in the range of from 400 to 800 MPa measured according to ISO 178, and a wall thickness in the range of from 0.3 to 0.7 mm.

The interruption of the grip recess serves for the defined introduction of force by the thumb or ball of the hand of the user. This helps dosing the force and thus the amount of irrigation solution applied. In particular in combination with the mounted bottle cap, an application-appropriate emptying of the container is made possible despite its grip stability.

Hence, in a first preferred embodiment of the present invention, the container is suitable for direct irrigation application and at least one diameter of the lower top area (**4**) and the middle bottom area (**7**) have the same length and/or at least one diameter of the upper bottom area (**11**) and the middle bottom area (**7**) have the same length.

Figure 4 also shows a container having the basic dimensions of a container according to Figure 2. The difference to the container of Figure 2 is the circumferentially formed recessed grip, which, in contrast to the partially formed grip geometry as shown in Figure 3, brings even more rigidity to the container. Such a shape is particularly advantageous for larger format containers, i.e., containers having an inner cavity volume of more than 500 ml. Figure 3 also shows a container with a screw cap. Such a feature is frequently used for indirect rinsing.

Hence, in the second preferred embodiment of the present invention, the container is suitable for indirect irrigation application, wherein the main axes of the lower top area (**4**) and the middle bottom area (**7**) are parallel and in a projection of the middle bottom area (**7**) on the lower top area (**4**) each diameter of the lower top area (**4**) is larger than the respective diameter in the same direction of the middle bottom area (**7**) and/or wherein the main axes of the upper bottom area (**11**) and the middle top area (**8**) are parallel and in a projection of the middle top area (**8**) on the upper bottom area (**11**) each diameter of the upper bottom area (**11**) is larger than the respective diameter in the same direction of the middle top area (**8**).

### Use

The present invention is further concerned with the use of the container according to the present invention in an irrigation application. In particular, the present invention is concerned with the use of the container of the present invention in an indirect and/or direct irrigation application.

### Process

Finally, the present invention is concerned with a process for producing a container according to the present invention, wherein the process comprises the steps of molding, preferably blow molding, the container, filling the container with an irrigation liquid, closing the container, and sterilizing the container.

## Claims

1. A container for irrigation application comprising
- a bottom part (**1**),
- a cylindrical or conical lower body part (**2**) comprising a lower bottom area (**3**) and a lower top area (**4**),
- a first transition part (**5**),
- a cylindrical or conical middle body part (**6**) comprising a middle bottom area (**7**) and middle top area (**8**),
- a second transition part (**9**), and
- a cylindrical or conical upper body part (**10**) comprising an upper bottom area (**11**),
wherein the bottom part (**1**) is fluidly connected with the lower body part (**2**) via the lower bottom area (**3**), the first transition part (**5**) is fluidly connected with the lower body part (**2**) via the lower top area (**4**), the first transition part (**5**) is fluidly connected with the middle body part (**6**) via the middle bottom area (**7**), the second transition part (**9**) is fluidly connected with the middle body part (**6**) via the middle top area (**8**), and the second transition part (**9**) is fluidly connected with the upper body part (**10**) via the upper bottom area (**11**),
wherein the lower bottom area (**3**), the lower top area (**4**), the middle bottom area (**7**), the middle top area (**8**), and the upper bottom area (**11**) have an oval shape,and
wherein the surface area of the middle bottom area (**7**) is lower than the surface area of the lower top area (**4**) and wherein the surface area of the middle top area (**8**) is lower than the surface area of the upper bottom area (**11**).

2. The container according to claim 1, wherein the container without the first transition part (**5**), without the cylindrical or conical middle body part (**6**), and/or without the second transition part (**9**) is a collapsible container.

3. The container according to claims 1 or 2, wherein the shape of the lower body part (**2**), the middle body part (**6**), and/or the upper body part (**9**) is cylindrical or conical.

4. The container according to any of the preceding claims, wherein the first transition part (**5**) is a cone, wherein in the cross section of the cone the shape of the rotational surface of the cone can be described by an asymmetric sigmoid function, wherein the change of slope in the curved area of the sigmoid function (a), which is closer to the lower body part (2), is smaller than the change of slope in the curved area of the asymmetric sigmoid function (b), which is closer to the middle body part (6).

5. The container according to any of the preceding claims, wherein the second transition part (9) is a cone, wherein in the cross section of the cone the shape of the rotational surface of the cone can be described by an asymmetric sigmoid function, wherein the change of slope in the curved area of the sigmoid function (**c**), which is closer to the upper body part (**10**), is smaller than the change of slope in the curved area of the asymmetric sigmoid function (**d**), which is closer to the middle body part (**6**).

6. The container according to any of the preceding claims, wherein the shape of the middle body part (**6**) is cylindrical.

7. The container according to any of the preceding claims, wherein the shape of the lower body part (**2**) and/or the upper body part (**10**) is cylindrical.

8. The container according to any of the preceding claims, wherein the lower bottom area (**3**), the lower top area (**4**), and the upper bottom area (**11**) have the same shape.

9. The container according to any of the preceding claims, wherein the oval shape is a shape selected from the list consisting of an ellipsoid, a rectangle with quadrant corners, and a stadium, preferably is a stadium shape.

10. The container according to any of the preceding claims, wherein the height of the middle body part (**6**) is lower than the height of the lower body part (**2**), the height of the middle body part (**6**) is lower than the height of the upper body part (**10**), and/or the height of the lower body part (**2**) is larger than the height of the upper body part (**11**).

11. The container according to any of the preceding claims, wherein the symmetry axis of the lower bottom area (**3**), the lower top area (**4**), the middle bottom area (**7**), the middle top area (**8**), and/or the upper bottom area (**11**) are parallel, preferably identical.

12. The container according to any of the preceding claims, wherein the upper body part comprises at least one outlet (**12**), preferably selected from the list consisting of a tear-off opening, a spout, a twist-off opening, and a non-return valve.

13. The container according to any of the preceding claims, wherein the material of the container comprises, preferably consists of, a polymer material preferably a polymer material selected from the list consisting of polyethylene, polypropylene, polyethylene terephthalate, polyamide, ethylene vinyl alcohol, or mixture thereof.

14. The use of the container according to any of the preceding claims in an irrigation application.

15. A process for producing a container according to any of claims 1 to 13, the process comprising the steps of molding, preferably blow molding, the container, filling the container with an irrigation liquid, closing the container, and sterilizing the container.
